# EUROPEAN PATENT APPLICATION

(11) **EP 3 124 498 A1**
(43) Date of publication of application: **01.02.2017**
(21) Application number: 14887280.7
(22) Date of filing: 27.08.2014
(51) Int. Cl.: C07K 16/14, G01N 33/53

(54) **AFLATOXIN B1 NANOBODY 2014AFB-G15**

(30) Priority: 28.03.2014 CN 201410121842
(71) Applicant: Oilcrops Research Institute of Chinese Academy of Agriculture Sciences, Wuhan, Hubei 430062 (CN)
(72) Inventor: LI, Peiwu, Wuhan Hubei 430062 (CN); WANG, Yanru, Wuhan Hubei 430062 (CN); ZHANG, Qi, Wuhan Hubei 430062 (CN); ZHANG, Zhaowei, Wuhan Hubei 430062 (CN); DING, Xiaoxia, Wuhan Hubei 430062 (CN)
(74) Representative: Laufhütte, Dieter
(86) International application number: PCT/CN2014/085313
(87) International publication number: WO 2015/143833

(57) **Abstract**

Disclosed is aflatoxin B1 nanobody 2014AFB-G15. An amino acid sequence thereof is shown in SEQ ID NO: 7, and a gene coding sequence thereof is shown in SEQ ID NO: 8. Aflatoxin B1 nanobody 2014AFB-G15 obtained in the present disclosure has the properties of resistance to organic reagents, resistance to high temperature, and good stability. 50% inhibitory concentration IC₅₀ of aflatoxin B1 nanobody 2014AFB-G15 against aflatoxin B1 is 0.66 ng/mL, and cross-reaction rates thereof with aflatoxin B2, aflatoxin G1, aflatoxin G2, and aflatoxin M1 are 22.6%, 0.95%, 32.1%, and 26%, respectively.

## Description

### Technical Field

The present disclosure relates to aflatoxin B1 nanobody 2014AFB-G15.

### Technical Background

Aflatoxins, secondary metabolites mainly generated by *Aspergillus flavus* and *Aspergillus parasiticus,* are natural toxic compounds that can cause damage to human and animals. More than twenty types of aflatoxins have been discovered so far. Among them, aflatoxin B1 is considered the most toxic, and its toxicity is 10 times that of potassium cyanide and 68 times that of arsenic. Early in 1993, aflatoxin B1 was listed, by the International Agency for Research on Cancer of World Health Organization, as one of the most carcinogenic substances known, belonging to group I in the classification of possible carcinogens. China is one of the regions that are seriously contaminated by aflatoxins which are likely to be found in various foods and farm products, especially in corn, peanuts, and products thereof. Consequently, it is of great significance to reinforce the detection, especially quick detection of aflatoxins, and to keep track of health information about foods and farm products so as to guarantee food safety in China.

Existing methods for detecting aflatoxins include chemical analysis method, instrumental analysis method, and immunoassay method. Among these methods, chemical analysis method is the most commonly used method for detecting aflatoxins in early times. Chemical analysis method does not need any special instrument or equipment, and can be performed in ordinary laboratories, but it has disadvantages such as large amounts of reagent consumption, complex operation, serious interference by other components, poor accuracy, incapability of accurate quantification, and great contamination to experimenters and the surroundings. And more importantly, it is not applicable to on-site quick detection. Instrumental analysis method involves fluorospectrophotometry and high performance liquid chromatography, and has advantages of high sensitivity and good accuracy, but it requires expensive instruments, highly purified aflatoxin samples, complex sample pretreatment, high standard experiment environment, and is time-consuming and incapable of achieving a quick detection. Immunoassay technology is developed in recently years, and it overcomes the defects of the above two methods. It has advantages such as strong specificity, high sensitivity, simple sample pretreatment, low cost, less contamination to the experimenters and the surroundings, applicability of on-site batch detection, etc. Immunoassay is a test used to conduct qualitative and quantitative detection for ultramicro-residues by utilizing specific binding of an antigen and an antibody, as well as the biological, physical, or chemical magnification of a marker on the antigen and the antibody. In order to develop an immunological technology with respect to aflatoxins, antibodies to aflatoxins must be prepared first.

With the development of antibody technologies, recombinant antibodies have been gradually used in the technical field of detecting aflatoxins. Compared with traditional polyclonal antibodies and monoclonal antibodies, recombinant antibodies have unique advantages. For example, recombinant antibodies can be mass-produced in procaryotic expression system in a short time and at low cost, which is greatly valued in the application of low-cost and large-scale detection of aflatoxins, and can satisfy the increasing demand for the production of aflatoxin antibodies. A nanobody is a natural heavy chain antibody variable region fragment obtained in camelids by means of molecular biology methods, and can be specifically bound to an antigen. So far, there has not yet been any report on aflatoxin B1 nanobodies.

### Summary of the Invention

The objective of the present disclosure is to provide aflatoxin B1 nanobody 2014AFB-G15.

In order to achieve the above objective, the present disclosure adopts the following technical solutions.

An aflatoxin nanobody gene library is obtained by extracting RNA from the blood of an alpaca immunized with aflatoxin B1 antigen; specifically amplifying IgG2 and IgG3 heavy chain antibodies variable region genes of the alpaca, namely, VHH genes, by RT-PCR; and then ligating the amplified genes with vector pCANTAB 5E (his); and transforming.

In the above technical solution, the vector pCANTAB 5E (his) is constructed through the following method: using pCANTAB5E vector plasmid as a template, p5E SfiI-F: 5'-ATGCGGCCCAGCCGGCC-3'(Sfi I) as a forward primer and p5E N-P-H-R: 5'- GATCGGGCCCTGTGGTGGTGGTGGTGGTGTGCG GCCGCCCGTTTTC-3' as a reverse primer, PCR amplifying a DNA fragment between *Sfi* I and *Not* I on the vector pCANTAB 5E (his) by PCR so as to obtain p5E-his fragments; then, subjecting the p5E-his fragments to single *Sfi* I restriction endonuclease digestion followed by single PspoMI restriction endonuclease digestion to obtain p5E-his(*Sfi* I/PspoMI) cohesive termini, and subjecting the pCANTAB5E vector plasmid to single *Sfi*I restriction endonuclease digestion followed by single PspoMI restriction endonuclease digestion to obtain p5E (*Sfi* I/*Not* I) cohesive termini; and finally, ligating the p5E-his(*Sfi* I/PspoMI) cohesive termini with the p5E (*Sfi* I/*Not* I) cohesive termini to obtain the vector pCANTAB 5E (his).

The aflatoxin nanobody gene library is constructed by the following method: immunizing an alpaca with aflatoxin B1 antigen; extracting RNA from the blood of the immunized alpaca; reversely transcribing the extracted RNA into cDNA; designing specific primers and using cDNA as a template to obtain IgG2 and IgG3 heavy chain antibodies variable region genes in the blood of the alpaca, namely, VHH gene, by PCR amplification; and then ligating the VHH genes with the vector pCANTAB 5E (his) followed by transformation to construct the aflatoxin nanobody gene library.

In the above technical solution, termini of the specific primer should contain primer sequences designed based on sequence nearby cloning sites of the vector pCANTAB 5E (his), for introducing homologous sequence of the vector pCANTAB 5E (his) at both termini of the VHH gene. The termini of each of the specific primers contain at least 15 bp of homologous sites of the vector pCANTAB 5E (his), for introducing at least 15 bp of homologous sequences of the vector pCANTAB 5E (his) at both termini of the VHH gene. The specific primers are:
R1: 5'-CGGCGCACCTGCGGCCGCATGGGGGTCTTCGCTGTGGTG CG -3'
F: 5-TCCTTTCTATGCGGCCCAGCCGGCCATGGCCC CAGKTGCAGCTCGTGGAGTC-3'; or
R2: 5'-CGGCGCACCTGCGGCCGCGTCTTGTGGTTTTGGTGTCTTG GG -3'
F: 5'-TCCTTTCTATGCGGCCCAGCCGGCCATGGCCC CAGKTGCAGCTCGTGGAGTC-3',
wherein the underlined primer sequences represent sites that are homogenous with the vector pCANTAB 5E (his).

In the above technical solution, a reaction system of the PCR amplification comprises:

| | |
|---|---|
| 10×Extaq Buffer | 5 µl |
| 50 mM MgSO₄ | 2 µl |
| 10 mMdNTP | 1 µl |
| 10 mM primier F | 1 µl |
| 10 mM primer R1 (or primer R2) | 1 µl |
| Ex taq DNA polymerase | 0.1 µl |
| cDNA template | 2 µl |
| ddH₂O added up to the total system | 50 µl. |

Procedures of the PCR amplification are: 94°C for 2 min, 94°C for 30 s, 55°C for 30 s, 68°C for 1 min, amplification for 30 cycles, 68°C for 5 min, wherein a ratio of number of the PCR amplification using R1 as the primer to number of the PCR amplification using R2 as the primer is 2:3.

In the above technical solution, the VHH genes are ligated into the vector pCANTAB 5E (his) by digesting the vector pCANTAB 5E (his) with double enzyme using *Sfi* I/*Not* I, and then ligating the vector pCANTAB 5E (his) with the VHH genes by In-fusion technology. The transformation is performed by: adding the product resulting from the aforesaid ligation to electrotransformation-competent cells of *Escherichia coli* TG1; mixing uniformly; performing electrotransformation using a 0.1 cm electroporation cuvette under conditions of 1.8 kV, 200 Ω, and 25 µF, followed by immediately adding 2YT liquid medium into the electroporation cuvette, pipetting up and down, transferring, and then reviving cells at 37°C for 1 h by slow shaking.

Application of the above aflatoxin nanobody gene library in screening aflatoxin B1 nanobodies by phage display method is provided.

In the above technical solution, the screening of aflatoxin B1 nanobodies by phage display method can be achieved through the following technical solution: rescuing the successfully-constructed aflatoxin nanobody gene library with M13KO7 helper phage in a form of displaying it on the capsid surface of the phage; panning by absorption-elution method to screen positive wells that are able to specifically bind aflatoxin B1 and are unable to bind carrier protein BSA; then detecting culture solution of the positive wells screened in the first step using indirect competitive enzyme-linked immunosorbent assay (ELISA), wherein aflatoxin B1 is used as a competitive antigen; selecting clones with relatively high sensitivity; and finally screening to obtain phage-displayed aflatoxin B1 nanobodies.

An amino acid sequence of aflatoxin B1 nanobody 2014AFB-G15 is shown in SEQ ID NO: 7, and a gene coding sequence thereof is shown in SEQ ID NO: 8. Amino acid sequences of three complementary determining regions are an amino acid sequence of a complementary determining region CDR1 shown in SEQ ID NO: 1, an amino acid sequence of a complementary determining region CDR2 shown in SEQ ID NO: 2, and an amino acid sequence of a complementary determining region CDR3 shown in SEQ ID NO: 3, respectively. Gene coding sequences of the three complementary determining regions are a gene coding sequence of CDR1 as depicted in SEQ ID NO: 4, a gene coding sequence of CDR2 as depicted in SEQ ID NO: 5, and a gene coding sequence of CDR3 as depicted in SEQ ID NO: 6, respectively.

Application of the above aflatoxin B1 nanobody 2014AFB-G15 in measuring aflatoxin B1 by ELISA is provided.

The present disclosure brings the following beneficial effects.
(1) The construction of the aflatoxin nanobody gene library provided by the present disclosure is simple, and aflatoxin B1 nanobody 2014AFB-G15 obtained through the screening has the properties of resistance to organic reagents, resistance to high temperature, and good stability.
(2) The 50% inhibitory concentration IC₅₀ of aflatoxin B1 nanobody 2014AFB-G15 provided by the present disclosure against aflatoxin B1 is 0.66 ng/mL, and the cross reaction rates with aflatoxin B2, aflatoxin G1, aflatoxin G2, and aflatoxin M1 are 22.6%, 0.95%, 32.1%, and 26%, respectively.
(3) Aflatoxin B1 nanobody 2014AFB-G15 provided by the present disclosure can be applied in detection of aflatoxin B1 by ELISA, and can effectively reduce interferences by other components such as organic reagents in the sample extraction solution, thus improving the accuracy of detection.
(4) Aflatoxin B1 nanobody 2014AFB-G15 provided by the present disclosure is expressed by prokaryote such as *Escherichia coli,* and therefore the production cost of the nanobody can be reduced.

### Detailed Description of the Embodiments

### Example 1

### Construction of an Aflatoxin Nanobody Gene Library

### 1. Immunization of Animal

One two-year old male alpaca was purchased and immunized with aflatoxin B1 antigen (AFB₁-BSA, Sigma-Aldrich Corporation). 200 µg of aflatoxin B1 antigen was emulsified with Freund's adjuvant incomplete, and was injected subcutaneously into the alpaca at multiple sites. The alpaca was immunized at an interval of 2 weeks. Blood was sampled intravenously from the alpaca 7-10 days after each immunization, and serum titer was determined by indirect ELISA. After selection of an immunization with the highest titer, 10 mL of blood was sampled for extracting total RNA.

### 2. Construction of a cDNA Library

(1) Extraction of total RNA was achieved by: selecting an immunization with the highest serum titer in the alpaca, and 7-10 days after an immunization, intravenously sampling 10 mL of blood from the alpaca for extracting total RNA. The total RNA was extracted from the blood of the alpaca using LeukoLOCK Total RNA Isolation System of Life Technologies.
(2) Synthesis of cDNA was performed by: using the total RNA obtained in step (1) as a template and oligo (dT)₁₅ as a primer, and performing reverse transcription according to the reverse transcriptase instruction of Promega Inc. to synthesize the first strand cDNA to obtain the cDNA library.

### 3. Construction of an Aflatoxin Nanobody Gene Library

(1) PCR amplification was performed to obtain heavy chain antibodies variable region genes of the alpaca, i.e., VHH genes, by using the cDNA obtained in step (2) as a template, and R1, F or R2, F as primers, which specifically is: taking 2 µl of cDNA, 5 µl of 10×PCR Buffer, 2 µl of MgSO₄ (50 mM), 1 µl of dNTP (10 mmol/L), 1 µl of F primers (10 µmol/L), 1 µl of R1 (or R2) primer (10 µmol/L), 0.1 µl of DNAase, 37.9 µl to 50 µl of sterile purified water, vortex mixing, centrifuging briefly, and performing PCR amplification with reaction conditions of: denaturation at 94°C for 2 min, followed by 30 cycles of denaturation at 94°C for 30 s, annealing at 55°C for 30 s, and elongation at 68°C for 1 min; and then elongation at 68°C for 5 min.
The primers were:
R1: 5'-CGGCGCACCTGCGGCCGCATGGGGGTCTTCGCTGTG GTGCG -3',
R2: 5'-CGGCGCACCTGCGGCCGCGTCTTGTGGTTTTGGTGTCTTG GG -3',
F: 5'-TCCTTTCTATGCGGCCCAGCCGGCCATGGCCC CAGKTGCAGCTCGTGGAGTC-3';
wherein the underlined primer sequences represent sites that are homogenous with vector pCANTAB 5E (his). PCR amplification reactions were performed 4 times using R1, F as primers, and 6 times using R2, F as primers. PCR products were separated by 0.7% agarose gel electrophoresis, and 450 bp of DNA fragments were recovered using purification kit.
(2) The vector pCANTAB 5E (his) was constructed as follows: amplifying a DNA fragment between *Sfi* I and *Not* I on the plasmid of the vector pCANTAB5E by PCR using plasmid of the vector pCANTAB5E as a template, p5E SfiI-F: 5'-ATGCGGCCCAGCCGGCC-3'(*Sfi* I) as a forward primer and p5E N-P-H-R: 5'-GATCGGGCCCTGTGGTGGTGGTGGTGGTGTGCGCCGCCCGTTTTC-3' as a reverse primer to obtain p5E-his fragments; and then, digesting the p5E-his fragments first with *Sfi* I restriction enzyme and then with PspoMI restriction enzyme to obtain p5E-his(*Sfi* I/PspoMI) with cohesive termini, and digesting the plasmid of the vector pCANTAB5E first with *Sfi* I and then with *Not* I to obtain p5E (*Sfi* I/*Not* I) with cohesive termini; and finally, ligating the cohesive termini of p5E-his(*Sfi* I/PspoMI) with the cohesive termini of p5E (*Sfi* I/*Not* I) to obtain vector pCANTAB 5E (his).
(3) Double enzyme digestion treatment of pCANTAB 5 E (his)
Single enzyme digestion was performed using *Sfi* I, and reaction solution thereof was prepared according to the following system:

| | |
|---|---|
| pCANTAB 5 E (his) vector | 30 µl |
| *Sfi* I | 1 µl |
| 10×M Buffer | 10 µl |
| ddH₂O added up to the total system | 100 µl. |

The reaction solution was incubated in water bath at 50 °C for 2 h, followed by recovering the DNA fragment with agarose gel DNA purification kit.
Enzyme digestion was performed using *Not* I, and reaction solution thereof was prepared according to the following system:

**Product recovered from single enzyme digestion**

| | |
|---|---|
| of pCANTAB 5 E (his) using *Sfi* I | 30 µl |
| *Not* I | 1 µl |
| 10×H Buffer | 10 µl |
| ddH₂O added up to the total system | 100 µl. |

The reaction solution was incubated in water bath at 37° C for 4 h, followed by recovering the DNA fragment with agarose gel DNA purification kit.
(4) The VHH Genes were ligated with vector pCANTAB 5 E (his) treated by double enzyme digestion by using In-fusion technology according to the following system:

| | |
|---|---|
| pCANTAB 5 E (his) vector treated with *Sfi* I/*Not* I double enzyme digestion | 120 ng |
| VHH genes | 40 ng |
| 5×In-Fusion buffer | 2 µl |
| In-Fusion Enzyme | 1 µl |
| ddH₂O added up to the total system | 10 µl |

The prepared solution was incubated in water bath at 37° C for 15 min, incubated in water bath at 50° C for 15 min, then immediately placed onto ice and kept for 5 min, added with 40 µl of TE buffer, and finally recovered using agarose gel DNA purification kit, and stored at -20 °C for use.
(5) Electroporation of the ligation product was performed by the following steps. 5 µl of the above ligated product was added into 50 µl electroporation-competent cells of *E. coli* TG1 and mixed uniformly, and was then added into a prechilled 0.1 cm-gap electroporation cuvette (Bio-RAD), placed on ice and kept for 10 min, put on Bio-rad electroporator for electroporation under electroporation conditions of 1.8 kV, 200 Ω, and 25 µF, followed by immediately adding 1 mL of 2YT liquid medium into the electroporation cuvette, pipetting up and down, transferring the mixture into a sterilized clean shake tube having a volume of 15 mL, and incubating the cells at 37°C by slow shaking for 1 h. 2 µl of bacterial culture was subjected to serial dilution, and was plated onto LB-ampicillin plates. The plates were inverted and cultured at 37°C overnight. The next day, the library capacity was calculated by counting the number of bacterial colonies.
(6) Rescue of aflatoxin nanobody gene library was performed as follows. The above-mentioned electroporation was performed ten times in total. The revived bacterial culture was entirely transferred into 200 mL of SB culture medium, shaken at 37°C at a speed of 250 rpm until OD₆₀₀ was 0.5, and then was added with 1 mL of helper phage M13KO7 with 1×10¹² pfu. After being kept standing at 37°C for 1 h, the bacterial culture was shaken for 2 h, added with kanamycin to reach a final concentration of 70 µg/mL, and was shaken overnight. The next day, the overnight bacteria culture was centrifuged at a speed of 10,000 rpm for 15 min at 4°C.The supernatant was transferred into a sterile centrifuge bottle, added with 1/4 volume of 5×PEG/NaCl, kept standing on ice for 2 h, and centrifuged at a speed of 12,000 rpm for 20 min at 4°C. Pellet was dissolved in 10 mL sterile resuspension solution (PBS buffer containing 1×protease inhibitor, 0.02% NaN₃ and 0.5% BSA) to obtain the rescued aflatoxin nanobody gene library.

### Example 2

### Screening and Sequencing of AflatoxinB1 Nanobodies

### 1. Panning of AflatoxinB1 Nanobodies

ELISA plates were coated with AFB₁-BSA (1 µg/well) and 3% BSA-PBS solution (used as a negative control) respectively at 4°C overnight. The next day, the coating solutions were poured off. The plates were washed with PBST 3 times, and blocked with 3% skimmed milk powder for 1 h. The plates were washed with PBST 3 times, and 50 µl of the above-mentioned rescued aflatoxin nanobody gene library was added into each well coated with AFB₁-BSA, and then incubated at 37°C for 1 h. The plates were washed with PBST 10 times, and 100 µl of 100 ng/mL AFB₁ solution was added into each well. The plates were eluted by shaking at room temperature (20°C-30°C) for 30 min. The eluate was transferred to the wells coated with 3% BSA-PBS solution and the plates were incubated at 37°C for 1 h (to remove non-specific adsorption). After incubation, the supernatant was taken to infect 2 mL of TG1 bacterial culture which has grown to the logarithmic phase, and the infection was allowed at 37°C for 20 min. 1 µl and 10 µl of the infected bacterial culture were respectively taken and plated onto LB-ampicillin plates. The LB-ampicillin plates were kept standing in an incubator set at 37°C overnight, and the next day the phage titer in the eluate was determined by counting the number of the colonies on the plates. Additionally, the remaining above-mentioned infected TG1 bacterial culture was transferred into 6 mL of SB medium with 1.5 µl of 100 mg/mL ampicillin, shaken at 37 °C for 1 h, supplemented with ampicillin to reach a final concentration of 50 µg/mL, shaken for 1 h, added with 1 mL of helper phage M13KO7 (1×10¹²pfu/mL), and was kept standing at 37°C for 30 min. The bacterial culture was then transferred into 100 mL of SB medium, added with 46 µl of ampicillin (100 mg/mL), shaken for 2 h, supplemented with kanamycin to reach a final concentration of 70 µg/mL, and was shaken at 37°C overnight. The next day, the bacterial culture was centrifuged at a speed of 10,000 rpm for 15 min at 4°C. The supernatant was then transferred, and added with 1/4 volume of PEG/NaCl solution, incubated on ice for 2 h, centrifuged at a speed of 12,000 rpm for 20 min at 4°C. The pellet was dissolved with 1% BSA-PBS solution to obtain the amplified product of the first round of panning, which was used for the next round of panning. In the subsequent several rounds of panning, the concentrations of the coating antigen AFB₁-BSA were 0.5 µg/well, 0.1 µg/well, and 0.05 µg/well, respectively, and the eluents were AFB₁ solutions of 500 ng/mL, 100 ng/mL, and 50 ng/mL, respectively.

### 2. Identification of Positive Clones

After 4 rounds of panning, 2 µl of eluate was taken and subjected to series dilution to infect TG1 bacterial culture which has grown to the logarithmic phase. The infected TG1 bacterial culture was plated onto LB-ampicillin plates. The plates were placed upside down and incubated at 37°C overnight. The next day, 30 clones were randomly picked out, and each of the clones was separately cultured in 3 mL of SB-ampicillin culture medium at 37°C for 6-8 h. The resulting bacterial culture was shaken to be cultured until OD₆₀₀ was about 0.6, then added with 30 µl of helper phage M13KO7 (1×10¹² pfu/mL), and was kept standing at 37°C for 30 min, shaken for 2 h, supplemented with kanamycin to reach a final concentration of 70 µg/mL, and then shaken to be cultured overnight. The next day, the bacterial culture was centrifuged at s speed of 10,000 rpm for 15 min at 4°C to obtain the supernatant of the bacterial culture.

AFB₁-BSA was prepared by coating solution to reach a final concentration of 0.2 µg/ml. The prepared AFB₁-BSA was then used to coat 96-well ELISA plate, with 100 µl in each well. Meanwhile, another ELISA plate was taken, and 32 wells thereof were coated with 3% BSA at 4°C overnight. The next day, the coating solution was poured off, and the plates were washed with PBST for 3 times, and then blocked with 3% skimmed milk powder-PBS for 1 h. AFB₁ standard stock solution was taken and prepared into working solutions having concentrations of 100 ng/mL and 0 ng/mL using 10% methanol/PBS, which were respectively added into the wells coated with AFB₁-BSA antigens. Then, 50 µl of the above-mentioned supernatant of bacterial culture was added into each well. The assay with each concentration of the working solutions was repeated 3 times. 10% methanol/PBS and 50 µl of the above-mentioned supernatant of bacterial culture were added into the wells coated with BSA as controls, and mixed uniformly by slightly shaking the plates, and the plates were placed at a 37°C incubator to allow reaction for 1 h. The plates were washed with PBST 10 times, and 100 µl of HRP/ANTI-M13 diluted with PBS in a ratio of 1:5000 was added into each well, and the plates were incubated at 37°C for 1 h. The plates were washed with PBST 6 times, and 100 µl of freshly prepared TMB substrate solution was added into each well, and the plates were incubated at 37°C for 15 min. 50 µl of 2 mol/L H₂SO₄ was added into each well to terminate the reaction, and OD₄₅₀ values were measured by a plate reader. The positive phage clones were those that did not adsorb BSA, but adsorbed AFB₁-BSA, and were in competition with the addition of aflatoxin. The wells having both relatively high absorbance and sensitivity were chosen, thereby obtaining phage-displayed aflatoxin B1 nanobody 2014AFB-G15.

### 3. Results of Properties and Sequencing Analysis of Aflatoxin B1 Nanobody 2014AFB-G15 were as Follows.

The antibody specificity of aflatoxin B1 nanobody 2014AFB-G15, described particularly in cross reaction rate, was determined by indirect competitive ELISA method as follows. Five different standard stock solutions of AFB₁, AFB₁, AFG₁, AFG₂, and AFM₁ were diluted in gradient to ten different working concentrations by 10% methanol/PBS to determine the antibody specificity by the indirect competitive ELISA method under same conditions. Competitive ELISA curves were drawn sequentially for the five aflatoxins. The standard concentrations of the five aflatoxins which had 50% inhibition rates were calculated respectively, and were represented by IC₅₀. Cross reaction rates were then calculated based on the following formula: cross reaction rate (%)=(AFB₁ IC₅₀/analogue IC₅₀)×100%, wherein the analogue was AFB₂, AFG₁, AFG₂, or AFM₁. The 50% inhibiting concentration IC₅₀ of aflatoxin B1 nanobody 2014AFB-G15 against aflatoxin B1 was 0.66 ng/mL, and the cross reaction rates with aflatoxin B2, G1, G2, and M1 were 22.6%, 10.95%, 32.1%, and 26%, respectively. Therefore, aflatoxin B1 nanobody 2014AFB-G15 was a specific nanobody against aflatoxin B1. It can be known from tolerance tests that, compared with conventional murine source antibodies and rabbit source antibodies, the resistance of aflatoxin B1 nanobody 2014AFB-G15 against organic solvents was improved by 35%, and the resistance against high temperature thereof was improved by 46%. Consequently, aflatoxin B1 nanobody 2014AFB-G15 can effectively reduce the interference by other ingredients such as organic solvents in the sample extraction solution during detection, thereby improving the detection sensitivity.

Sequencing of the characteristic sequence of aflatoxin B1 nanobody 2014AFB-G15 was as follows. The screened clonal bacterial culture containing aflatoxin B1 nanobody 2014AFB-G15 was sent to Shanghai Sunny Biotechnology Co., Ltd. to conduct sequencing analysis. The sequencing primer was phage vector universal primer R1: 5'-CCA TGA TTA CGC CAA GCT TTG GAG CC-3'. The obtained amino acid sequence of aflatoxin B1 nanobody 2014AFB-G15 was shown in SEQ ID NO: 7, and the gene coding sequence thereof was shown in SEQ ID NO: 8. The amino acid sequences of three complementary determining regions were: the amino acid sequence of CDR1 shown in SEQ ID NO: 1, the amino acid sequence of CDR2 shown in SEQ ID NO: 2, and the amino acid sequence of CDR3 shown in SEQ ID NO: 3, respectively, and gene coding sequences of the three complementary determining regions were: the gene coding sequence of CDR1 shown in SEQ ID NO: 4, the gene coding sequence of CDR2 shown in SEQ ID NO: 5, and the gene coding sequence of CDR3 shown in SEQ ID NO: 6, respectively.

### Example 3

### Preparation of Aflatoxin B1 Nanobody 2014AFB-G15

(1) TG1 bacterial culture capable of secreting aflatoxin B1 nanobody 2014AFB-G15 was obtained, and DNA mini-extraction kit of Qiagen was used to extract plasmids. The extracted plasmids were transformed to HB2151 competent cells, and the transformed competent cells were plated onto LB-ampicillin plates.
(2) HB2151 colonies containing aflatoxin B1 nanobody 2014AFB-G15 plasmids were picked out and inoculated into 100 mL of SB-ampicillin liquid medium, and cultured at a speed of 250 rpm at 37° C until OD₆₀₀=0.5-0.8, then 200 µl of 0.5 M IPTG solution was added into the culture for induction overnight.
(3) The cultured medium after induction was centrifuged at a speed of 10,000 rpm for 15 min at 4°C. The supernatant was removed carefully in a sterile operation bench, and the soluble protein was extracted from the bacterial cell pellets by an osmotic shock method to obtain a supernatant containing the protein. The supernatant containing the protein was filtered by a 0.22 µm filter membrane, and dialyzed in an equilibrium buffer (50 mM phosphate, 300 mM sodium chloride, 20 mM imidazole, pH7.4) overnight.
(4) His60 nickel column (Clontech Technology) was used to purify antibody. Firstly, the nickel column was rinsed with an equilibrium buffer in 10 folds the column volume. The supernatant containing the protein dialyzed in step (3) was loaded to His60 nickel column (Clontech Technology) for antibody purification. Then, the column was washed with a rinsing buffer (50 mM phosphate, 300 mM sodium chloride, 40 mM imidazole, pH7.4) in 10 folds the column volume. Finally, antibody 2014AFB-G15 was eluted with an elution buffer (50 mM phosphate, 300 mM sodium chloride, 300 mM imidazole, pH7.4) in 10 folds the column volume. The eluate was collected and put into a dialysis bag, dialyzed using 0.01 M, pH7.4 phosphate buffer for 2-3 days and then concentrated, fractionized and stored at -20° C for use.

### Example 4

### Application of AflatoxinB1 Nanobody 2014AFB-G15

### 1. Sample Pretreatment

5 g of corn oil, 5 g of peanut oil, 5 g of plant oil, and 5 g of feed blank sample, having no aflatoxin, were weighed respectively in triplicate, and were added with standard AFB₁ having a concentration of 10 µg/kg, 50 µg/kg, and 100 µg/kg, respectively. The samples were subjected to ultrasonic extraction with 15 mL of 70% methanol aqueous solution at 50°C for 10 min. After the extracted samples have been kept standing for a while, the supernatants were obtained and diluted 5 folds with 0.01 M, pH 7.4 phosphate buffer for use.

### 2. Indirect Competitive Enzyme-Linked Immunosorbent Assay

AFB₁-BSA solution having a concentration of 0.25 µg/mL was prepared using a coating buffer, and was added into an ELISA micro well plate, 100 µL for each well. The plate was kept at 4°C overnight. The next day, the plate was washed with PBST 3 times, and the microwells were blocked with 200 µL of 3% skimmed milk powder soluble in PBS. The plate was placed into incubator set at 37°C to allow reaction for 1 h. The plate was washed with PBST 3 times, and 50 µL of aflatoxin B1 nanobody 2014AFB-G15 (1.26 µg/mL) and 50 µL of AFB₁ standard solution or sample to be tested were added into each well. Reaction was allowed at 37° C for 1 h. The plate was washed with PBST 3 times. 100 µL of rabbit anti-E tag enzyme-labelled secondary antibody diluted with PBS in a ratio of 1:5000 was added into each well, and reaction was performed at 37° C for 1 h. The plate was washed with PBST 6 times, and 100 µL of freshly-prepared TMB substrate solution was added into each well, and the plate was incubated at 37° C for 15 min. Then, 50 µL of 2 M sulphuric acid was added into each well to terminate the reaction, and the absorbance value at 450 nm was immediately measured using a plate reader. The concentration of AFB₁ in the samples was calculated based on the measured absorbance values of the samples, and recovery rates were determined. The average recovery rates of the corn oil, peanut oil, plant oil, and feed blank sample were 92.8%, 88.7%, 97.3%, and 90.2%, respectively, which can meet the requirements for accuracy of the method for detecting aflatoxins.

In summary, aflatoxin B1 nanobody 2014AFB-G15 can effectively recognize aflatoxin B1 and can be used in an ELISA for detecting aflatoxin B1, and it is of important significance for monitoring aflatoxins in the field of food safety.

## Claims

1. Aflatoxin B1 nanobody 2014AFB-G15, **characterized in that** : an amino acid sequence of aflatoxin B1 nanobody 2014AFB-G15 is shown in SEQ ID NO:7, and a gene coding sequence of aflatoxin B1 nanobody 2014AFB-G15 is shown in SEQ ID NO:8.

2. Aflatoxin B1 nanobody 2014AFB-G15 according to claim 1, **characterized in that**:
amino acid sequences of three complementary determining regions of aflatoxin B1 nanobody 2014AFB-G15 are: an amino acid sequence of a complementary determining region CDR1 shown in SEQ ID NO: 1, an amino acid sequence of a complementary determining region CDR2 shown in SEQ ID NO: 2, and an amino acid sequence of a complementary determining region CDR3 shown in SEQ ID NO: 3, respectively; and
gene coding sequences of the three complementary determining regions are: a gene coding sequence of the CDR1 shown in SEQ ID NO: 4, a gene coding sequence of the CDR2 shown in SEQ ID NO: 5, and a gene coding sequence of the CDR3 shown in SEQ ID NO: 6, respectively.

3. Application of aflatoxin B1 nanobody 2014AFB-G15 or 2 in an ELISA for detecting aflatoxin B 1.


SEQUENCE LISTING

<110>  Oil Crops Research Institute of Chinese Academy of
       Agriculture Science

<120>  AFLATOXIN B1 NANOBODY 2014AFB-G15

<150>  CN/2014101218422
<151>  2014-03-28

<160>  13

<210> 1
<211> 8
<212> PRT
<213> Vicugna vicugna

<400>1
Gly Arg Thr Phe Ser Ser Tyr Ala
1   5

<210> 2
<211> 7
<212> PRT
<213> Vicugna vicugna

<400>2
Ile Ser Trp Ser Gly Gly Ser
1  5

<210> 3
<211> 14
<212> PRT
<213> Vicugna vicugna

<400>3
Ala Ala Gly Phe Ser Gly Asn Tyr Tyr Arg Thr Pro Asp Tyr
1               5                   10              14

<210> 4
<211> 24
<212> DNA
<213> Vicugna vicugna

<400> 4
ggacgcacct tcagtagcta cgcc   24

<210> 5
<211> 21
<212> DNA
<213> Vicugna vicugna

<400> 5
attagctgga gtggtggtag c     21

<210> 6
<211> 42
<212> DNA
<213> Vicugna vicugna

<400> 6
gcagctggct ttagtggtaa ttactaccgc acacccgact ac    42

<210> 7
<211> 130
<212> PRT
<213> Vicugna vicugna

<400> 7
Gln Leu Gln Leu Val Glu Ser Gly Gly Gly Leu Val Gln Ala Gly
1               5                   10
15
Gly Ser Leu Arg Leu Ser Cys Ala Ala Ser Gly Arg Thr Phe Ser
                    20                  25
30
Ser Tyr Ala Met Gly Trp Phe Arg Gln Ala Pro Gly Lys Glu Arg
                35                  40
45
Glu Phe Val Ala Ala Ile Ser Trp Ser Gly Gly Ser Thr Tyr Tyr
            50                  55
60
Thr Asp Ser Val Lys Gly Arg Phe Thr Ile Asn Arg Asp Asn Ala
            65                  70
75
Lys Asn Thr Val Tyr Leu Gln Met Asn Ser Leu Lys Pro Glu Asp
            80                      85
90
Thr Ala Val Tyr Tyr Cys Ala Ala Gly Phe Ser Gly Asn Tyr Tyr
                95                  100
105
Arg Thr Pro Asp Tyr Trp Gly Gln Gly Thr Gln Val Thr Val Ser
                110                 115
120
Ser Glu Pro Lys Thr Pro Lys Pro Gln Asp
                125                 130

<210> 8
<211> 390
<212> DNA
<213> Vicugna vicugna

<400> 8
cagttgcagc tcgtggagtc tgggggagga ttggtgcagg ctgggggctc tctgagactc   60
tcctgtgcag cctctggacg caccttcagt agctacgcca tgggctggtt ccgccaggct   120
ccagggaagg agcgtgagtt tgtagcggct attagctgga gtggtggtag cacatactat   180
acagactccg tgaagggccg attcaccatc aacagagaca acgccaagaa cacggtgtat  240
ctgcaaatga acagcctgaa acctgaggac acggccgttt attactgtgc agctggcttt    300
agtggtaatt actaccgcac acccgactac tggggccagg ggacccaggt caccgtctcc  360
tcagaaccca agacaccaaa accacaagac
390

<210>  9
<211>  17
<212>  DNA
<213>  Artificial Sequence

<220>
<223>  Primer

<400>  9
atgcggccca gccggcc                                                17

<210>  10

<211>  46
<212>  DNA
<213>  Artificial Sequence

<220>
<223>  Primer

<400>  10
gatcgggccc tgtggtggtg gtggtggtgt gcggccgccc gttttc                    46


<210>  11
<211>  41
<212>  DNA
<213>  Artificial Sequence

<220>
<223>  Primer

<400>  11
cggcgcacct gcggccgcat gggggtcttc gctgtggtgc g                         41


<210>  12
<211>  52
<212>  DNA
<213>  Artificial Sequence

<220>
<223>  Primer

<400>  12
tcctttctat gcggcccagc cggccatggc cccagktgca gctcgtggag tc             52


<210>  13
<211>  42
<212>  DNA
<213>  Artificial Sequence

<220>
<223>  Primer

<400>  13
cggcgcacct gcggccgcgt cttgtggttt tggtgtcttg gg                        42